(19) Europäisches Patentamt
European Patent Office
Office européen des brevets

(11) **EP 4 438 027 A1**

(12) **EUROPEAN PATENT APPLICATION**
published in accordance with Art. 153(4) EPC

(43) Date of publication:
**02.10.2024 Bulletin 2024/40**

(21) Application number: **22968573.0**

(22) Date of filing: **16.12.2022**

(51) International Patent Classification (IPC):
*A61K 6/802* (2020.01)    *A61C 5/70* (2017.01)
*A61C 13/083* (2006.01)    *C04B 35/111* (2006.01)

(52) Cooperative Patent Classification (CPC):
**A61C 5/70; A61C 13/083; A61K 6/802;
C04B 35/111**

(86) International application number:
**PCT/JP2022/046499**

(87) International publication number:
**WO 2024/127666 (20.06.2024 Gazette 2024/25)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Kuraray Noritake Dental Inc.
Kurashiki-shi, Okayama 710-0801 (JP)**

(72) Inventors:
• **KATO Shinichiro
Miyoshi-shi, Aichi 470-0293 (JP)**

• **SAKAMOTO Hiroyuki
Miyoshi-shi, Aichi 470-0293 (JP)**
• **NAKANO Kirihiro
Miyoshi-shi, Aichi 470-0293 (JP)**
• **KASHIKI Nobusuke
Miyoshi-shi, Aichi 470-0293 (JP)**
• **ISHINO Hiroshige
Miyoshi-shi, Aichi 470-0293 (JP)**

(74) Representative: **D Young & Co LLP
3 Noble Street
London EC2V 7BQ (GB)**

(54) **ALUMINA WORKABLE BODY FOR DENTAL USE**

(57)     The present invention provides an alumina workable body for dental use that exhibits excellent translucency, strength, and linear light transmittance as a sintered body after sintering. The present invention relates to an alumina workable body for dental use with D1 and D2 that are different from each other, where D1 is the post-sintering average crystal grain size at a first point falling within an interval from one end P to 25% of the entire length on a straight line extending along a first direction from said one end P to the other end Q of the alumina workable body for dental use, and D2 is the post-sintering average crystal grain size at a second point falling within an interval from said other end Q to 25% of the entire length.

FIG.1

**Description**

TECHNICAL FIELD

The present invention relates to an alumina workable body for dental use. More specifically, the present invention relates to an alumina workable body for dental use that exhibits excellent translucency, strength, and linear light transmittance as a sintered body after sintering.

BACKGROUND ART

Traditionally, metals have been commonly used for dental prostheses (for example, such as veneer crowns, dental caps, crowns, and post crowns). However, metals have the drawback of lacking aesthetics, and occasionally causing allergic reactions due to leaching of metal. To address the issues associated with the use of metals, ceramic materials such as lithium disilicate glass and zirconium oxide (stabilized zirconia) have been used for dental products as alternatives to metal. Zirconia, in particular, offers superior aesthetics and strength, and has seen a rise in demand, especially due to its increasing affordability in recent years.

] To achieve improved aesthetics in the oral cavity, it is necessary for dental prostheses to mimic the look of natural teeth. However, achieving a natural tooth-like appearance, especially in terms of transparency, gloss (glaze or shine), and shade, is difficult with zirconia (a sintered body) alone.

Techniques are available that involve stacking multiple layers containing oxide pigments mixed with stabilized zirconia (Patent Literatures 1 and 2), as well as techniques involving stacking multiple layers with varying amounts of stabilizing agent (Patent Literature 3).

Research has also been conducted on the particles of sintered aluminum oxide (alumina) as base material ceramic, with discussions on the quantity of particles larger than 1 $\mu$m in the sintered particles, and the contrast ratio of the sintered body. There are also reports of high-transparency, high-strength alumina as dental cutting materials (Patent Literatures 4 and 5).

CITATION LIST

Patent Literature

Patent Literature 1: JP 2017-185163 A
Patent Literature 2: WO2019/131782
Patent Literature 3: WO2020/138316
Patent Literature 4: JP 2005-514305 T
Patent Literature 5: JP 2001-213664 A

SUMMARY OF INVENTION

Technical Problem

Typically, dental prostheses are created by applying a slurry containing feldspar glass materials, which transform into porcelain, onto base material ceramic, followed by firing at temperatures of several hundred degrees Celsius to fuse the porcelain to the base material. To prevent defects during firing, it is therefore required to choose a porcelain material with a coefficient of thermal expansion similar to that of the base material. For example, when a zirconia sintered body is used as the base material, it is essential to choose a porcelain ceramic material with a coefficient of thermal expansion close to that of the zirconia sintered body.

Additionally, in the process of PFZ fabrication, it has been required to heat the porcelain to a temperature at which it melts (typically ranging from about 900 to 1,000°C) using an electric furnace, after applying a porcelain slurry to the zirconia surface. There accordingly is a need for a more convenient method to produce prostheses with high aesthetics without using porcelain.

The methods described in Patent Literatures 1 and 2 involve layering zirconia raw materials of different shades to vary the values of $\Delta L^*$, $a^*$, $b^*$ for each layer from top to bottom, with the aim of providing a gradation of shades. Patent Literature 3 aims to provide a gradation of translucency by layering raw materials with varying amounts of stabilizing agent (yttria) to vary the $\Delta L^*$ value for each layer from top to bottom.

However, achieving aesthetically pleasing results with the translucency obtained from zirconia has not been satisfactory. This is due to the refractive index effect of zirconia, causing surfaces to exhibit glare-like reflection similar to metal, necessitating the coating of the surface with a porcelain material having a different refractive index.

Patent Literature 4 describes particle sizes after sintering, featuring an average particle diameter of 1.0 micron or less, and 10% or smaller proportions of particles having a particle size larger than 1.0 micron in maximum diameter.

However, this related art does not address the technical advantages of these features, nor does it describe methods for deliberately controlling particle size.

Patent Literature 5 describes compositions involving highly transparent alumina. However, there is no examination of layered structures aimed at reproducing the shade, which is crucial for dental prostheses, presenting numerous challenges for dental applications.

It is accordingly an object of the present invention to provide an alumina workable body for dental use that exhibits excellent translucency, strength, and linear light transmittance as a sintered body after sintering.

Solution to Problem

The present inventors focused on the crystal grain size of alumina sintered bodies to overcome the foregoing issues, and found that, by varying the crystal grain size, it is possible to provide the same or similar variation seen in the translucency of natural teeth while ensuring the required level of high translucency exhibited by natural teeth, allowing for achieving a perception of translucency comparable to natural teeth. This led to the completion of the present invention after further examinations.

Specifically, the present invention relates to the following.

[1] An alumina workable body for dental use with D1 and D2 that are different from each other, where D1 is the post-sintering average crystal grain size at a first point falling within an interval from one end P to 25% of the entire length on a straight line extending along a first direction from said one end P to the other end Q of the alumina workable body for dental use, and D2 is the post-sintering average crystal grain size at a second point falling within an interval from said other end Q to 25% of the entire length.

[2] The alumina workable body for dental use according to [1], wherein at least two of the D1, D2, and D3 are different when D3 is the post-sintering average crystal grain size at a third point falling between the first point and the second point on a straight line extending along the first direction from one end P to the other end Q of the alumina workable body for dental use.

[3] The alumina workable body for dental use according to [1] or [2], which shows an unchanging pattern of increase or decrease in post-sintering average crystal grain size from one end P to the other end Q of the alumina workable body for dental use on a straight line extending along the first direction from one end P to the other end Q of the alumina workable body for dental use.

[4] The alumina workable body for dental use according to any one of [1] to [3], wherein the D1 is 0.3 $\mu$m or more and 3.0 $\mu$m or less.

[5] The alumina workable body for dental use according to any one of [1] to [4], wherein the D2 is 1.0 $\mu$m or more and 8.0 $\mu$m or less.

[6] The alumina workable body for dental use according to any one of [1] to [5], wherein the difference between the D1 and the D2 is 0.3 $\mu$m or more.

[7] The alumina workable body for dental use according to any one of [1] to [6], which has a linear light transmittance of 0.8% or more and a biaxial flexural strength of 200 MPa or more at the first point after sintering.

[8] The alumina workable body for dental use according to any one of [1] to [7], which has a linear light transmittance of 6.0% or less and a biaxial flexural strength of 400 MPa or more at the second point after sintering.

[9] The alumina workable body for dental use according to any one of [1] to [8], which comprises a sintering aid at the first point.

[10] The alumina workable body for dental use according to any one of [1] to [9], wherein the content of the sintering aid is different at the first point and the second point.

[11] The alumina workable body for dental use according to any one of [1] to [10], wherein the content of the sintering aid shows an unchanging pattern of increase or decrease from said one end P to said other end Q.

[12] The alumina workable body for dental use according to any one of [9] to [11], wherein the sintering aid comprises at least one element selected from the group consisting of a Group 2 element, Ce, Zr, and Y

[13] The alumina workable body for dental use according to [12], wherein the Group 2 element is Mg.

[14] The alumina workable body for dental use according to any one of [1] to [13], wherein the content of the sintering aid is 30 ppm or more and 3,000 ppm or less at the first point.

[15] The alumina workable body for dental use according to any one of [1] to [14], wherein the content of the sintering aid is 0 ppm or more and 2,000 ppm or less at the second point.

[16] The alumina workable body for dental use according to any one of [1] to [15], which comprises a plurality of layers with different average crystal grain sizes after sintering.

[17] The alumina workable body for dental use according to [16], wherein the plurality of layers has the smallest post-sintering average crystal grain size in the layer including said one end P.

[18] The alumina workable body for dental use according to [16] or [17], wherein the plurality of layers has the largest post-sintering average crystal grain size in the layer including said other end Q.

[19] The alumina workable body for dental use according to any one of [1] to [18], wherein the alumina workable body for dental use is an alumina pre-sintered body.

[20] The alumina workable body for dental use according to any one of [1] to [19], which comprises alumina particles with an average roundness of 0.81 or more in primary particles of the alumina pre-sintered body.

Advantageous Effects of Invention

According to the present invention, an alumina workable body for dental use can be provided that exhibits excellent translucency, strength, and linear light transmittance as a sintered body after sintering.

The present invention can also provide an alumina sintered body for dental use that exhibits high strength, and translucency and refractive index similar to those of natural teeth, as well as high linear light transmittance, without using specialized sintering equipment (for example, such as hot isostatic pressing (HIP) machines). An alumina workable body for dental use for fabricating such alumina sintered bodies for dental use can also be provided. Particularly, the present invention can provide a dental prosthesis through processing of such alumina workable bodies for dental use, achieving an appearance close to natural teeth, including the incisal region, where transparency exhibits variation depending on the location, with high linear light transmittance in portions corresponding to the incisal region, and low linear light transmittance in portions corresponding to the cervical region.

In prostheses made of zirconia, the surface tends to exhibit glare-like reflection similar to metal due to the effect of the zirconia's refractive index, necessitating the coating of the surface with a porcelain material having a different refractive index. However, according to the present invention, an alumina workable body for dental use can be provided that can minimize the requirement for porcelain coating because the alumina workable body, in the form of a sintered body, shows an appearance that more closely resembles natural teeth compared to zirconia due to the difference in refractive index from zirconia prostheses.

The present invention can also provide an alumina workable body for dental use that exhibits excellent total transmittance.

The present invention can also provide an alumina workable body for dental use that exhibits excellent polishability.

BRIEF DESCRIPTION OF DRAWINGS

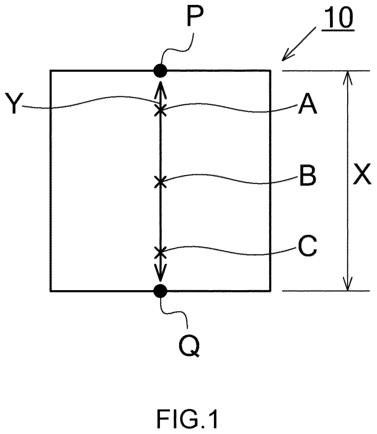

[FIG. 1] A schematic view of an alumina workable body for dental use.

DESCRIPTION OF EMBODIMENTS

An alumina workable body for dental use of the present invention is described below with reference to the schematic view depicted in FIG. 1.

The alumina workable body for dental use 10 depicted in FIG. 1 has D1 and D2 that are different from each other, where D1 is the post-sintering average crystal grain size at a first point A falling within an interval from one end P to 25% of the entire length X on a straight line extending along a first direction Y from said one end P to the other end Q of the alumina workable body for dental use 10, and D2 is the post-sintering average crystal grain size at a second point C falling within an interval from said other end Q to 25% of the entire length X.

In the present invention, the method of measurement of the post-sintering average crystal grain size at each point is as described in the EXAMPLES section below.

By using a predetermined alumina workable body, the present invention can reproduce a refractive index closer to that of natural teeth compared to zirconia, preventing glare-like surface reflection similar to metal while ensuring the high strength required for dental prostheses.

Because such glare-like surface reflection is attributed to zirconia, it has been difficult with zirconia sintered bodies to reduce excessive reflections while providing excellent translucency such as linear light transmittance.

By setting different average crystal grain sizes for the first point corresponding to the incisal region (or "incisal edge" as it is also called) and the second point corresponding to the cervical region of dental prostheses, it is possible to set the appropriate translucency for these locations, enabling the recreation of appropriate natural teeth aesthetics.

It is preferable in an alumina workable body for dental use of the present invention that D1, D2, and D3 be all different when D3 is the post-sintering average crystal grain size at a third point B falling between the first point A and the second point C on a straight line extending along the first direction Y from one end P to the other end Q of the alumina workable body for dental use.

By setting the appropriate average crystal grain size for the third point corresponding to the intermediate layer, in addition to the first point corresponding to the incisal region and the second point corresponding to the cervical region of dental prostheses, it is possible to more closely reproduce the location-dependent behavior of translucency variation seen in natural teeth (for example, a gradation of shades with no visible layer boundaries).

More precise control over translucency is possible when additional points with different post-sintering average crystal grain sizes are set between the first, second, and third points.

A certain preferred embodiment is, for example, an alumina workable body for dental use in which D1 and D2 are different when D1 is the post-sintering average crystal grain size at a first point falling within an interval from one end P to 25% of the entire length on a straight line extending along a first direction from said one end P to the other end Q of the alumina workable body for dental use, and D2 is the post-sintering average crystal grain size at a second point falling within an interval from said other end Q to 25% of the entire length, and in which at least two of the D1, D2, and D3 are different when D3 is the post-sintering average crystal grain size at a third point falling between the first point and the second point on a straight line extending along the first direction from one end P to the other end Q of the alumina workable body for dental use.

An example of such an embodiment is an alumina workable body for dental use in which D1 and D2 are different, and D2 and D3 are the same among D1, D2, and D3. Specifically, D1, D2, and D3 preferably satisfy $D1 < D3 \leq D2$.

Another certain preferred embodiment is, for example, an alumina workable body for dental use in which the D1, D2, and D3 are all different.

An example of such an embodiment is an alumina workable body for dental use satisfying $D1 < D3 < D2$.

In view of achieving translucency and strength suited as a dental prosthesis after sintering, as well as providing superior linear light transmittance, it is preferable that an alumina workable body for dental use of the present invention show an unchanging pattern of increase or decrease in post-sintering average crystal grain size from one end P to the other end Q of the alumina workable body for dental use on a straight line extending along the first direction from one end P to the other end Q of the alumina workable body for dental use. In other words, it is preferable that the post-sintering average crystal grain size monotonously increase or decrease on a straight line extending along the first direction.

Preferably, the pattern of increase or decrease in post-sintering average crystal grain size does not change in the opposite direction on a straight line extending along the first direction Y from one end P to the other end Q of the alumina workable body for dental use 10 depicted in FIG. 1. Specifically, when the post-sintering average crystal grain size is in a pattern of increase on a straight line from one end P toward the other end Q, it is preferable that there exist no interval in which the post-sintering average crystal grain size essentially decreases. To describe further, when the post-sintering average crystal grain size is in a pattern of increase on a straight line from one end P toward the other end Q, there may exist an interval in which the average crystal grain size shows essentially no change.

In view of achieving translucency suited for the incisal region of dental prostheses, particularly, high linear light transmittance suited for the incisal region, an alumina workable body for dental use of the present invention has a D1 of preferably 0.3 $\mu$m or more, more preferably 0.5 $\mu$m or more, even more preferably 0.7 $\mu$m or more. Preferably, D1 is 3.0 $\mu$m or less, more preferably 2.5 $\mu$m or less, even more preferably 2.0 $\mu$m or less.

In view of achieving translucency suited for the cervical region of dental prostheses, particularly linear light transmittance suited for the cervical region, an alumina workable body for dental use of the present invention has a D2 of preferably 1.0 $\mu$m or more, more preferably 1.4 $\mu$m or more, even more preferably 2.0 $\mu$m or more. D2 is preferably 8.0 $\mu$m or less, more preferably 6.0 $\mu$m or less, even more preferably 4.0 $\mu$m or less, particularly preferably 3.0 $\mu$m or less.

In view of achieving a closer resemblance to natural teeth with D1 and D2 by providing an intermediate portion between the incisal region and cervical region of dental prostheses to allow for gradual changes in translucency and linear light transmittance with no boundary between the incisal region and cervical region, an alumina workable body for dental use of the present invention has a D3 of preferably 0.5 $\mu$m or more, more preferably 0.6 $\mu$m or more, even more preferably 0.8 $\mu$m or more. D3 is preferably 8.0 $\mu$m or less, more preferably 6.0 $\mu$m or less, even more preferably 4.0 $\mu$m or less, particularly preferably 3.0 $\mu$m or less.

In view of reproducing the appropriate translucency difference between the incisal region and cervical region of dental prostheses, it is preferable in an alumina workable body for dental use of the present invention that the difference between D1 and D2 be 0.3 $\mu$m or more, more preferably 0.5 $\mu$m or more, even more preferably 0.7 $\mu$m or more. In view of more closely reproducing the behavior of translucency and linear light transmittance seen in natural teeth, D1 is preferably smaller than D2 (D1/D2 < 1). In view of reproducing the appropriate translucency difference between the incisal region and cervical region of dental prostheses, the difference between D1 and D2 is preferably 5.0 $\mu$m or less, more preferably 3.5 $\mu$m or less, even more preferably 2.5 $\mu$m or less.

In view of reproducing the appropriate translucency difference between the incisal region and cervical region of dental prostheses, it is preferable in an alumina workable body for dental use of the present invention that the difference between D3 and D1 be 0.1 $\mu$m or more, more preferably 0.2 $\mu$m or more, even more preferably 0.3 $\mu$m or more. In view of more closely reproducing the behavior of translucency and linear light transmittance seen in natural teeth, D1 is preferably smaller than D3. The difference between D3 and D1 is preferably 5.0 $\mu$m or less, more preferably 3.5 $\mu$m or less, even more preferably 2.5 $\mu$m or less.

In view of more closely reproducing the location-dependent behavior of translucency variation seen in natural teeth, it is preferable in an alumina workable body for dental use of the present invention that the difference between D3 and D2 be 0 $\mu$m or more, more preferably 0.1 $\mu$m or more, even more preferably 0.2 $\mu$m or more. In view of more closely reproducing the behavior of translucency and linear light transmittance seen in natural teeth, D3 is preferably less than or equal to D2. The difference between D3 and D2 is preferably 4.0 $\mu$m or less, more preferably 3.0 $\mu$m or less, even more preferably 2.0 $\mu$m or less.

An alumina workable body for dental use of the present invention has a linear light transmittance of preferably 0.8% or more, more preferably 1.0% or more, even more preferably 2.0% or more at the first point after sintering.

In certain preferred embodiments, the linear light transmittance at the first point after sintering may be 2.5% or more, 3.5% or more, 5.0% or more, 5.5% or more, 6.0% or more, or 6.5% or more for the incisal region of dental prostheses, according to the patient's preferences.

Regarding the linear light transmittance at the first point after sintering, the upper limit is not particularly limited. However, in terms of providing superior aesthetics for the incisal region of dental prostheses, the upper limit is preferably 50.0% or less, more preferably 30.0% or less, even more preferably 20.0% or less, particularly preferably 15.0% or less, most preferably 12.0% or less.

When the linear light transmittance at the first point after sintering is less than 0.8%, it may not be possible to achieve the translucency (transmissivity for linear light) required for the incisal region of dental prostheses.

In the present invention, the method of measurement of linear light transmittance at each point after sintering is as described in the EXAMPLES section below.

The biaxial flexural strength at the first point after sintering is preferably 200 MPa or more, more preferably 300 MPa or more, even more preferably 400 MPa or more. When the biaxial flexural strength at the first point after sintering is less than 200 MPa, it may not be possible to ensure the strength required for the incisal region of dental prostheses, potentially leading to the occurrence of defects such as fractures or cracks. In the present invention, the method of measurement of biaxial flexural strength at each point after sintering is as described in the EXAMPLES section below.

An alumina workable body for dental use of the present invention has a linear light transmittance of preferably 6.0% or less, more preferably 4.0% or less, even more preferably 2.0% or less, particularly preferably 1.5% or less at the second point after sintering. When the linear light transmittance at the second point after sintering is more than 6.0%, it leads to an increased translucency (transmissivity for linear light) in the cervical region of dental prostheses, and it may not be possible to sufficiently mask the abutment teeth, even with adjustments using materials such as colorants.

The biaxial flexural strength at the second point after sintering is preferably 400 MPa or more, more preferably 500 MPa or more, even more preferably 600 MPa or more. When the biaxial flexural strength at the second point after sintering is less than 400 MPa, it may not be possible to ensure the strength required for the cervical region of dental prostheses, potentially leading to the occurrence of defects such as fractures or cracks.

In an alumina workable body for dental use of the present invention, it is preferable that the linear light transmittance at the first point after sintering be higher than the linear light transmittance at the second point after sintering. More preferably, the linear light transmittance at the first point is higher than the linear light transmittance at the second point by 0.3% or more, more preferably by 0.5% or more, even more preferably by 0.7% or more after sintering.

In view of providing high translucency, particularly, high linear light transmittance after sintering, an alumina workable body for dental use of the present invention preferably comprises a sintering aid (an auxiliary agent that accelerates and stabilizes sintering of alumina) at the first point.

In view of allowing for adjustments that lead to different D1 and D2 values, and providing the appropriate translucency for the incisal region and cervical region of dental prostheses after sintering, it is preferable for the sintering aid content to differ between the first point and the second point. More preferably, the sintering aid content at the first point is higher than the sintering aid content at the second point. The sintering aid may be absent at the second point.

In view of achieving translucency and strength suited as a dental prosthesis after sintering, it is preferable that an alumina workable body for dental use of the present invention show an unchanging pattern of increase or decrease in sintering aid content from one end P to the other end Q on a straight line extending along the first direction from one end P to the other end Q of the alumina workable body for dental use. In other words, it is preferable that the sintering aid content monotonously increase or decrease.

This is described below with reference to FIG. 1 representing a schematic view of an alumina workable body for dental use.

Preferably, the pattern of increase or decrease in sintering aid content does not change in the opposite direction on a straight line extending along the first direction Y from one end P to the other end Q of the alumina workable body for dental use 10 depicted in FIG. 1. Specifically, when the sintering aid content is in a pattern of decrease on a straight line from one end P toward the other end Q, it is preferable that there exist no interval in which the sintering aid content essentially increases.

To describe further, when the sintering aid content is in a pattern of decrease on a straight line from one end P toward the other end Q, there may exist an interval in which the sintering aid content shows essentially no change.

The sintering aid contained in an alumina workable body for dental use of the present invention comprises preferably at least one element selected from the group consisting of a Group 2 element (Be, Mg, Ca, Sr, Ba, Ra), Ce, Zr, and Y, more preferably at least one element selected from the group consisting of Mg, Ca, Sr, Ba, Ce, Zr, and Y, even more preferably at least one element selected from the group consisting of Mg, Ce, Zr, and Y

The sintering aid may be used alone, or two or more thereof may be used in combination.

In view of providing translucency, particularly, high linear light transmittance for the dental prosthesis, the Group 2 element is most preferably Mg (magnesium).

Examples of the magnesium compound include oxides, nitrates, acetates, hydroxides, and chlorides. The magnesium compound is not limited, as long as it is a magnesium compound that turns into an oxide at 1,200°C or less during a sintering process in the atmosphere. Preferred examples include magnesium chloride, magnesium hydroxide, magnesium nitrate, and magnesium acetate.

Examples of the sintering aid include $MgCl_2$, $Mg(OH)_2$, $CeO_2$, $ZrO_2$, and $Y_2O_3$.

In view of achieving translucency and strength suited for the incisal region of dental prostheses, the sintering aid content at the first point is preferably 30 ppm or more, more preferably 100 ppm or more, even more preferably 300 ppm or more in an alumina workable body for dental use of the present invention.

The sintering aid content at the first point is preferably 3,000 ppm or less, more preferably 2,500 ppm or less, even more preferably 2,000 ppm or less.

When the sintering aid content at the first point is less than 30 ppm, it leads to excessively low translucency, resulting in the inability to reproduce translucency suited for the incisal region of dental prostheses. When the sintering aid content at the first point exceeds 3,000 ppm, it may result in a sintered body with a yellow or red tint. This may result in the inability to reproduce shades suited for the incisal region of dental prostheses, and/or the failure to ensure the strength required for the incisal region of dental prostheses, potentially leading to the occurrence of defects such as fractures or cracks. The sintering aid content can be expressed as an amount in terms of the constituent element of the sintering aid (for example, in terms of a Mg element) relative to alumina.

In this specification, ppm means ppm by mass. The sintering aid content in the raw material alumina powder is the same as the sintering aid content in the alumina workable body for dental use.

In view of achieving translucency and strength suited for the cervical region of dental prostheses, the sintering aid content at the second point is preferably 0 ppm or more, more preferably 20 ppm or more, even more preferably 50 ppm or more in an alumina workable body for dental use of the present invention. The sintering aid content at the second point is preferably 2,000 ppm or less, more preferably 1,000 ppm or less, even more preferably 500 ppm or less. When the sintering aid content exceeds 2,000 ppm, it may not be possible to ensure the strength required for the cervical region of dental prostheses, potentially leading to the occurrence of defects such as fractures or cracks.

In an alumina workable body for dental use of the present invention, it is preferable that the sintering aid content at the first point be higher than the sintering aid content at the second point. More preferably, the sintering aid content at the first point is higher than the sintering aid content at the second point by 100 ppm or more, even more preferably by 200 ppm or more, particularly preferably by 300 ppm or more.

With the sintering aid content higher at the first point than at the second point, the post-sintering average crystal grain size can be more easily adjusted to the desired size to achieve different D1 and D2 values. In view of providing a post-sintering average crystal grain size within the desired range, the sintering aid content at the first point differs from the sintering aid content at the second point preferably by 3,000 ppm or less, more preferably by 2,500 ppm or less, even more preferably by 2,000 ppm or less.

It is preferable in an alumina workable body for dental use of the present invention that the sintering aid content at the first point be higher than the sintering aid content at the third point. More preferably, the sintering aid content at the first point is higher than the sintering aid content at the third point by 50 ppm or more, even more preferably by 100 ppm or more, particularly preferably by 150 ppm or more. With the sintering aid content higher at the first point than at the third point, the post-sintering average crystal grain size can be more easily adjusted to the desired size to achieve different D1 and D3 values.

It is preferable in an alumina workable body for dental use of the present invention that the sintering aid content at the third point be approximately equal to or higher than the sintering aid content at the second point. More preferably, the sintering aid content at the third point is higher than the sintering aid content at the second point by 10 ppm or more, even more preferably by 20 ppm or more, particularly preferably by 30 ppm or more. With the sintering aid content higher at the third point than at the second point, the post-sintering average crystal grain size can be more easily adjusted to the desired size to achieve the desired relationship between D3 and D2.

In an alumina workable body for dental use of the present invention, a layer with a different average crystal grain size can be additionally provided by setting another point (for example, a fourth point) with a different sintering aid content between the first point and the second point, in addition to the third point.

A certain preferred embodiment is, for example, an alumina workable body for dental use comprising a plurality of layers with different average crystal grain sizes after sintering. The number of layers is not particularly limited, as long as two or more layers are present, and may be 3, 4, or 5 or more. The plurality of layers comprises a layer including one end P, and a layer including the other end Q.

For convenience, the term "layers" in the plurality of layers refer to portions differing in sintering aid content during production, or portions with different average crystal grain sizes after the sintering and production of the alumina workable body for dental use. Accordingly, an alumina workable body for dental use of the present invention encompasses alumina workable bodies with no visually recognizable boundaries between layers. In view of aesthetics, it is not required for an alumina workable body for dental use of the present invention to have visually recognizable boundaries between layers after sintering. Preferably, an alumina workable body for dental use of the present invention has no visually recognizable boundaries between layers after sintering.

In view of reproducing translucency suited for the incisal region of dental prostheses, particularly, high linear light transmittance suited for the incisal region in the multiple layers with different post-sintering average crystal grain sizes, it is preferable that an alumina workable body for dental use of the present invention have the smallest post-sintering average crystal grain size in the layer containing one end P.

In view of reproducing translucency suited for the cervical region of dental prostheses in the multiple layers with different post-sintering average crystal grain sizes, it is preferable that an alumina workable body for dental use of the present invention have the largest post-sintering average crystal grain size in the layer containing other end Q.

The alumina raw material used for the fabrication of an alumina workable body for dental use of the present invention is not particularly limited. However, the alumina raw material is preferably aluminum oxide with a purity of 99.5% or more because of its low impurity content, and the ability to reduce the impurity-related formation of a glass phase at crystal grain boundaries, preventing coarsening of crystal grains, and inhibiting a decrease of aesthetic qualities in the sintered body as a dental material.

Using $\alpha$-phase aluminum oxide ($\alpha$-alumina) as the starting raw material is preferable because, with its high corrosiveness and high stability at elevated temperatures, it enables uniform control of the pre-sintered body, and a reduction of tool wear or chipping. Another reason is that it enables densification of crystal grains within the crystal structure in the sintered body, allowing for easier adjustment to the desired average crystal grain size with the sintering aid content, leading to superior translucency.

In view of these, the alumina raw material used for the fabrication of an alumina workable body for dental use of the present invention is particularly preferably $\alpha$-alumina with a purity of 99.5% or more.

The alumina raw material can be acquired using methods, for example, such as the alkoxide method, modified Bayer method, ammonium alum pyrolysis method, and ammonium dawsonite pyrolysis method, preferably the alkoxide method. The alkoxide method allows for enhancement of purity in a powder of alumina raw material, making it easier to achieve a uniform particle size distribution. As a specific example, aluminum hydroxide obtained through hydrolysis of purified aluminum alkoxide is fired in air at 1,100°C or higher temperatures.

In view of achieving translucency and strength suited for the incisal region of dental prostheses in a sintered body of the alumina workable body for dental use, the alumina raw material powder has an average primary particle diameter of preferably 30 to 300 nm. An alumina powder with an average primary particle diameter of 300 nm or less is preferable because the incorporation of smaller particles in the particle size distribution becomes less likely, reducing the occurrence of necking due to particle size differences, resulting in a reduction of the localized presence of coarse particles, and reduced chipping.

An alumina powder with an average primary particle diameter of 300 nm or less is also preferable because the post-sintering average crystal grain size does not overly increase, providing outstanding translucency and strength as dental materials.

An average primary particle diameter of 30 nm or more is preferable because it prevents an excessive increase in the number of necks between particles, inhibiting a hardness increase in the pre-sintered body, resulting in superior machinability, in the case of a pre-sintered body.

In view of allowing for easier adjustment of average crystal grain size to provide different D1 and D2 values when combined with other features such as differing sintering aid content at the first point and the second point, the alumina powder has an average primary particle diameter of more preferably 40 to 250 nm, even more preferably 60 to 200 nm, particularly preferably 80 to 180 nm.

The average primary particle diameter of the alumina powder can be measured using a laser diffraction scattering method. As an example of a laser diffraction scattering method, the average primary particle diameter can be measured by volume with ultrasonic waves being applied after a slurry diluted with water is subjected to 30 minutes of ultrasonication, using a laser diffraction/scattering particle size distribution analyzer (manufactured by Horiba Ltd. under the trade name Partica LA-950).

Examples of the alumina raw material with a purity of 99.5% or more include the NXA grade (ultrafine $\alpha$-

aluminawith a purity of 99.99% or more) manufactured by Sumitomo Chemical Co., Ltd., including NXA-100 (average primary particle diameter: 100 nm) and NXA-150 (average primary particle diameter: 150 nm).

An alumina workable body of the present invention may optionally comprise additives other than sintering aids (additives excluding $CeO_2$, $ZrO_2$, and $Y_2O_3$), such as colorants (including pigments, composite pigments, and fluorescent agents), titanium oxide ($TiO_2$), silica ($SiO_2$), dispersants, and antifoaming agents. These components may be used alone, or two or more thereof may be used as a mixture.

Examples of the pigments include oxides of at least one element selected from the group consisting of Ti, V, Cr, Mn, Fe, Co, Ni, Zn, Y, Zr, Sn, Sb, Bi, Ce, Sm, Eu, Gd, and Er.

Examples of the composite pigments include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$.

Examples of the fluorescent agents include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

An alumina workable body for dental use of the present invention may be any of an alumina molded body, an alumina pre-sintered body, and an alumina sintered body.

In view of processability, it is preferable in certain embodiments that the alumina workable body for dental use be an alumina molded body or alumina pre-sintered body, more preferably an alumina pre-sintered body.

In another embodiment, the alumina workable body for dental use may be an alumina sintered body.

The descriptions of the following embodiment are based on an example where an alumina workable body for dental use of the present invention is an alumina pre-sintered body.

The pre-sintered body can be a precursor (intermediate product) of a sintered body.

In this specification, "pre-sintered body" refers to a state where particles of alumina (hereinafter, also referred to as "alumina particles") have formed necks, and have solidified without being fully sintered.

The pre-sintered body may have a predetermined shape (a block shape, for example, such as a disc shape or cuboidal shape), or may be a workpiece that has been processed into a desired shape (for example, a crown shape).

In this specification, the pre-sintered body will be referred to as "workpiece" when it has been processed. The workpiece can be obtained by, for example, processing an alumina disc (representing an alumina workable body for dental use, for example, an alumina pre-sintered body) into a dental product (for example, a crown-shaped prosthesis) with the CAD/CAM (Computer-Aided Design/Computer-Aided Manufacturing) system.

An alumina pre-sintered body of the present invention can be obtained by molding an alumina composition as needed, and pre-sintering the alumina composition or molded body (pre-sintering step).

The alumina composition becomes a precursor of the alumina pre-sintered body.

In this specification, the alumina composition and molded body refer to a state before firing, and involve no necking between alumina particles.

The content of alumina and sintering aids in an alumina composition of the present invention is calculated from their content in a predetermined alumina pre-sintered body, and is the same for the alumina composition and alumina workable body for dental use (for example, the alumina pre-sintered body).

The alumina composition is not limited to particular forms, and an alumina composition of the present invention may have various forms, including a powder, a fluid with a powder added to solvent, and a molded body of a powder formed into a predetermined shape. When an alumina composition of the present invention has a powder form, it may be a cluster of granules. The granules are formed by the aggregation of primary particles.

In this specification, "primary particle" refers to a bulk representing the smallest unit. For example, "primary particle" refers to particles that appear spherical under an electron microscope (for example, a scanning electron microscope). The primary particles include alumina particles. When employing particulate sintering aids, the primary particles include particles of sintering aid, as well as alumina particles.

Preferably, the particles constituting the granules formed of the alumina composition are predominantly primary particles. Aggregates of primary particles are referred to as secondary particles. Preferably, the number of primary particles is greater than the number of secondary particles in visual confirmation through, for example, an electron microscope. Secondary particles are typically irregular in shape, and an increase in their quantity leads to sparse density during the press molding process described later, resulting in increased chipping.

The particle diameter of primary particles within the particles constituting the granules formed of the alumina composition influences the extent of necking during pre-sintering, and affects the hardness of the pre-sintered body. Particles with an average primary particle diameter of less than 30 nm are not preferable because it results in strong necking, leading to a decrease in the surface area of the primary particles contained in the pre-sintered body, and a hardness increase. Particles with an average primary particle diameter of more than 300 nm are not preferable because the incorporation of smaller particles in the particle size distribution becomes more likely, causing localized necking due to particle size differences, thereby increasing the occurrence of coarse density. The average primary particle diameter is preferably 30 to 300 nm, more preferably 40 to 250 nm, even more preferably 60 to 200 nm.

The primary particles within the particles constituting the granules formed of the alumina composition may be a combination of two types of alumina particles having different average primary particle diameters. For example, when the NXA is used, an example is a combination of NXA-100 and NXA-150.

The particles constituting the granules formed of the alumina composition has a BET specific surface area of preferably 5 $m^2$/g or more, more preferably 7.5 $m^2$/g or more, even more preferably 8 $m^2$/g or more, as measured in compliance with JIS Z 8830:2013.

With a BET specific surface area of 5 $m^2$/g or more, it is easier to lower the highest sintering temperature. This facilitates easier sintering, or makes it easier to inhibit the reduction in translucency due to the opacification occurring in the sintered body after sintering.

The BET specific surface area is preferably 25 $m^2$/g or less, more preferably 20 $m^2$/g or less, even more preferably 15 $m^2$/g or less.

A BET specific surface area of 25 $m^2$/g or less is preferable because it means that the average primary particle diameter is not excessively small, preventing the pre-sintered body from becoming overly hard, leading to a shorter polishing time and/or facilitating a reduction in chipping rate during polishing, resulting in a greater reduction in surface roughness Ra and/or Rz. Another reason is that it can reduce the occurrence of coarse density by ensuring sufficient necking, facilitating a further reduction in chipping rate during the polishing of the pre-sintered body, resulting in a greater reduction in surface roughness Ra and/or Rz.

An alumina composition of the present invention may employ a granular form in 50% or more, preferably 70% or more, more preferably 80% or more, even more preferably 90% or more of the alumina in the alumina composition.

When an alumina composition of the present invention does not employ a granular form, the alumina particles constituting the powder may have the average primary particle diameter and the BET specific surface area noted above.

The average particle diameter of granules in an alumina composition of the present invention (secondary particle diameter; hereinafter, also referred to as "average granule diameter") is preferably 10 $\mu$m or more, more preferably 12 $\mu$m or more, even more preferably 14 $\mu$m or more.

When the average granule diameter is less than 10 $\mu$m, there is a risk of trapping air in the process of placing the granules in a die, leading to inadequate degassing during molding. This may result in the inability to fabricate a uniform and dense molded body. Additionally, there is a risk of granules being ejected through gaps during molding, potentially resulting in a molded body that does not meet the predetermined quantitative requirements.

The average granule diameter is preferably 200 $\mu$m or less, more preferably 190 $\mu$m or less, even more preferably 180 $\mu$m or less, particularly preferably 150 $\mu$m or less, most preferably 100 $\mu$m or less.

When the average granule diameter exceeds 200 $\mu$m, there is an increased likelihood of cavity formation within the granules. Additionally, voids are more likely to occur when placing the granules in a die. These phenomena pose a risk of inadequate degassing during molding, potentially resulting in the inability to fabricate a dense molded body. There is also a risk of increased shrinkage during molding, potentially leading to the inability to fabricate a molded body of the desired size.

It is preferable that 50% or more of the alumina in the alumina composition constitute granules. Preferably, the average granule diameter is measured using a method that does not disrupt the granules. The average granule diameter can be measured using a method, for example, such as dry sieving or wet sieving.

Measurement by dry sieving can be conducted following the test sieving described in JIS Z 8815:1994. Both manual sieving and mechanical sieving are applicable; however, mechanical sieving is preferred.

For sieving, the test sieve described in JIS Z 8801-1:2019 can be used.

Ro-Tap sieve shakers or sonic sieving analyzers may be used as measurement instruments for sieving, for example. Examples of the Ro-Tap sieve shakers include RPS-105M manufactured by Seishin Enterprise Co., Ltd. Examples of the sonic sieving analyzers include Robot Sifter RPS-01 and Robot Sifter RPS-02 manufactured by Seishin Enterprise Co., Ltd.

It is preferable that granules in an alumina composition of the present invention have high sphericity. By increasing the sphericity of granules, it is possible to promote mixing at the interface between layers when stacking alumina powders of different compositions.

With increased sphericity, it is also possible to increase the packing density of when an alumina powder is filled into a mold to prepare a molded body, even when the average particle diameter is the same. The strength and translucency of the sintered body can increase by increasing the packing density, which is a density of a molded body formed into a specific shape under pressure after alumina granules are filled into a specific mold (such as a die).

It is also possible to more easily fill granules into angular portions when the mold has angular portions.

The sphericity of granules in an alumina composition of the present invention can be represented by parameters, for example, such as light bulk density, and heavy bulk density.

In view of increasing granule flow (ease of loading) to reduce coarseness in the density of the molded body obtained, an alumina composition of the present invention has a light bulk density of preferably 0.6 g/cm$^3$ or more, more preferably 0.7 g/cm$^3$ or more, even more preferably 0.8 g/cm$^3$ or more, particularly preferably 0.9 g/cm$^3$ or more.

The light bulk density can be measured in compliance with JIS R 9301-2-3:1999.

In view of increasing granule flow (ease of loading) to reduce coarseness in the density of the molded body obtained, an alumina composition of the present invention has a heavy bulk density of preferably 0.8 $g/cm^3$ or more, more preferably 0.9 $g/cm^3$ or more, even more preferably 1.0 $g/cm^3$ or more.

The heavy bulk density can be measured in compliance with JIS R 9301-2-3:1999.

An alumina composition of the present invention preferably comprises a binder.

The binder may be, for example, an organic binder. Examples of the organic binder include common binders such as acrylic binders, acrylate binders, paraffinic binders, fatty acid binders, and polyvinyl alcohol binders. Preferred among these organic binders is one having a carboxyl group in the molecular chain, or a derivative of carboxylic acids, more preferably an acrylic binder, even more preferably a polyacrylate having water solubility.

The polyacrylate may be a product of copolymerization of acrylic acid or methacrylic acid with maleic acid, and may contain sulfonic acid. Examples of the cations of the salt include sodium and ammonium.

Depending on the content of the binder in an alumina composition of the present invention, it is possible to adjust the distance between primary particles in the alumina composition, and the cumulative pore distribution and relative density, making it easier to adjust the Vickers hardness or the strength of the pre-sintered body by increasing or decreasing these factors.

The binder content is preferably 1.2 to 2.8 mass%, more preferably 1.5 to 2.5 mass%, even more preferably 1.8 to 2.2 mass% in the whole alumina composition. When the binder content is 1.2 mass% or more in the whole alumina composition, the pre-sintered body does not become overly strong, eliminating the risk of hardening when removing the workpiece.

When the binder content is 2.8 mass% or less, the strength of the pre-sintered body does not overly decrease, reducing the possibility of the workpiece detaching during cutting, in addition to facilitating a reduction in chipping rate.

An alumina composition of the present invention may comprise the additives described in conjunction with the alumina workable body. The additives may be added during mixing or pulverization, or may be added after pulverization.

The additives are as described for the additives of the alumina workable body (excluding the sintering aids).

An example of an alumina pre-sintered body producing method comprises the steps of:

producing a plurality of alumina compositions comprising alumina particles and a sintering aid and differing in sintering aid content; and
firing (pre-sintering) the alumina compositions (for example, a molded body) to obtain an alumina pre-sintered body. The alumina particles and sintering aid are as described for the alumina workable body for dental use.

First, alumina and a sintering aid are mixed in predetermined proportions to prepare a mixture (mixing step). For example, when the sintering aid is magnesium chloride, alumina and magnesium chloride may be mixed at a mixing ratio that provides the foregoing contents. The mixing process may be dry mixing or wet mixing. In view of enabling adjustment to the desired average roundness and the desired average crystal grain size after sintering, the alumina composition may be pulverized (preferably, crushed) until the foregoing average primary particle diameter is achieved (pulverization step).

The mixing and pulverization may be performed in a single step. Pulverization can be performed by using, for example, a ball mill or bead mill after dispersing the composition and binder in a solvent such as water or alcohol (dispersing step). In view of enabling the production of an alumina composition that is adjustable to achieve the desired average crystal grain size after sintering, the composition is pulverized (preferably, crushed) to achieve a particle size of, for example, 30 to 300 nm. For particle size adjustment, the composition may optionally be subjected to other processes (classification, elutriation).

After the mixing and/or pulverization step, the mixture may be dried by spray drying with a spray dryer or the like to achieve the aforementioned granular form in the alumina composition (drying step).

In the pulverization step, the average primary particle diameter of the alumina composition is preferably less than 0.3 $\mu$m, more preferably 0.25 $\mu$m or less, even more preferably 0.2 $\mu$m or less, particularly preferably 0.15 $\mu$m or less. When the average primary particle diameter of the alumina composition is less than 0.15 $\mu$m, it is possible to enhance the translucency of the sintered body after sintering while improving the machinability of the pre-sintered body.

Alumina and sintering aids may be separately prepared. For example, it is possible to independently provide separate preparation steps (for example, production steps) for alumina and sintering aids, instead of simultaneously precipitating alumina and sintering aids (in the same step). In this way, the aforementioned $\alpha$-aluminacan be obtained with high purity and a small primary particle diameter.

Typically, in the production of alumina compositions, sintering aids may be reacted with alumina through heat treatment, and the resulting product may be used for the pulverization and drying steps.

In the production of the alumina composition, the content of the sintering aid in the alumina composition constituting each layer is adjusted to provide a different sintering aid content for each layer of the laminate.

This allows for easier adjustment to the desired post-sintering average crystal grain size, facilitating even easier adjustment of average crystal grain size with the average primary particle diameter of the alumina powder.

This enables the production of granules formed of the alumina composition, which serves as the raw material of alumina pre-sintered bodies.

The granule or powder can be formed into a molded body by applying an external force. The molding method is not limited to specific methods, and a suitable method may be appropriately selected according to intended use. For example, molding may be achieved by press molding, injection molding, stereolithography, slip casting, gel casting, filtration-casting, or casting. Molding may be performed in multiple stages. For example, the alumina composition may be subjected to a CIP process after press molding, or press molding and CIP molding may be repeated.

Examples of the press molding method include a uniaxial pressing process (hereinafter, also referred to as "uniaxial pressing"), a biaxial pressing process, and a CIP (Cold Isostatic Pressing) process. These may be performed in combination, as needed.

A molded body of the present invention may have a disc shape, a cuboidal shape, or a shape of a dental product (for example, a crown shape).

A certain embodiment is, for example, a production method of an oxide ceramic pre-sintered body for dental use in which the press molding is uniaxial pressing, and the surface pressure of uniaxial pressing is 5 to 600 MPa. The press molding may yield, for example, a columnar molded body packed by uniaxially pressing alumina granules filled into a die. The molded body can increase its density with higher surface pressure during press molding. This leads to a greater relative density in the alumina pre-sintered body produced, while allowing for adjustment of average roundness. Conversely, a hard alumina pre-sintered body results when the density of the molded body is excessively high. The surface pressure in press molding is preferably 5 to 600 MPa, more preferably 10 to 400 MPa, even more preferably 15 to 200 MPa. When the surface pressure of pressing (for example, uniaxial pressing) is 5 MPa or more, the molded body exhibits superior shape retention. With a surface pressure of 600 MPa or less, it is possible to prevent excessive density increase in the molded body, making it easier to avoid hardening.

A molded body of the present invention encompasses molded bodies compacted by a high-temperature pressing process such as a CIP (Cold Isostatic Pressing) process. In view of the above, the hydraulic pressure is preferably 50 to 1,000 MPa, more preferably 100 to 600 MPa, even more preferably 150 to 300 MPa.

The content of the alumina and sintering aids in an alumina pre-sintered body of the present invention is the same as that in the alumina composition to be fabricated into an alumina pre-sintered body.

The following describes the step of firing (pre-sintering) the molded body under atmospheric pressure (pre-sintering step).

The firing temperature (hereinafter, also referred to as "pre-sintering temperature") in the pre-sintering step for producing an alumina pre-sintered body of the present invention influences the average roundness of particles contained in the pre-sintered body, and affects the Vickers hardness, or the strength of the pre-sintered body. The pre-sintered body undergoes changes in polishability and hardness with the pre-sintering temperature (highest pre-sintering temperature).

When the particles contained in the pre-sintered body have small average primary particle diameters, necking occurs at lower temperatures, increasing the Vickers hardness, or the strength of the pre-sintered body. For example, the pre-sintering temperature (highest pre-sintering temperature) is preferably around 750°C (700 to 850°C) when the average primary particle diameter of the particles contained in the pre-sintered body is 95 nm.

A pre-sintering temperature of 1,200°C or more for such an average primary particle diameter is not preferable because the average roundness decreases due to the formation of necks between particles, resulting in excessive hardness and a prolonged polishing time.

In a certain preferred embodiment, the highest pre-sintering temperature is preferably 400°C or more and less than 1,200°C, more preferably 700°C or more and less than 1,200°C, even more preferably 750°C or more and less than 1,200°C.

When the particles contained in the pre-sintered body have large average primary particle diameters, necking does not occur at lower temperatures but takes place at higher temperatures, increasing the Vickers hardness, or the strength of the pre-sintered body. For example, the pre-sintering temperature is preferably around 1,150°C (1,100°C or more and less than 1,200°C) when the average primary particle diameter of the particles contained in the pre-sintered body is about 280 nm.

A pre-sintering temperature of 1,000°C or less for such an average primary particle diameter is not preferable because the average roundness does not increase, and necking between particles does not proceed, failing to increase the strength of the pre-sintered body or Vickers hardness, causing chipping during polishing, and a reduction of surface roughness.

In another certain preferred embodiment, the highest pre-sintering temperature is preferably 1,000°C or more and less than 1,200°C, more preferably 1,050°C or more and less than 1,200°C, even more preferably 1,100°C or more and less than 1,200°C.

Concerning the average primary particle diameter of the particles contained in the pre-sintered body, it is the same as the average primary particle diameter of the alumina raw material powder described above.

The average primary particle diameter of the particles contained in the pre-sintered body can be measured using the following method, for example.

The pre-sintered body is used to prepare surface images, using a scanning electron microscope (VE-9800 manufactured by Keyence under this trade name). For the measurement of particle diameter, the SEM image with indicated primary particles is binarized with image analysis software (Image-Pro Plus manufactured by Hakuto Co., Ltd. under this trade name), and particles are recognized from the field (region) after indicating grain boundaries on individual crystal grains in the image. In areas with unclear grain boundaries, a reduce filter is applied to the region until each region reduces to a single or multiple points, and Voronoi polygons are created by using these points as generators of Voronoi polygons. Lines are drawn that connect the midpoints between two adjacent generators, and these lines are superimposed on the original particle image to separate adjacent particles. For example, in the case where image processing produces a particle that looks like a gourd, the particle is separated into two by assuming that two adjoining circular particles are seen as a single particle. In a processing file recognizing primary particle diameter, "Diameter" is selected from "Count/size dialog" to find the distribution (for example, n = 4). Specifically, a mean value of particle diameters (primary particle diameters) measured in each field with the image analysis software (Image-Pro Plus) is determined for four fields from one sample.

An alumina pre-sintered body of the present invention is a state where the alumina particles (powder) have not fully sintered, and, with necks formed between particles, undergoes changes in polishability according to the average roundness of the particles.

The polishability increases when the alumina particles contained in the pre-sintered body have an average roundness of 0.81 or more, leading to a decrease in surface roughness after polishing.

When the average roundness is less than 0.81, it leads to an increase in the surface roughness of the pre-sintered body after polishing, or results in a prolonged working time due to excessive hardness. The average roundness is preferably 0.82 or more, more preferably 0.83 or more, even more preferably 0.84 or more.

The average roundness can be measured using the following method, for example.

Using the data obtained from the measurement of the average primary particle diameter of particles contained in the pre-sintered body, the roundness calculated by the following formula is determined as average roundness.

$$\text{(Roundness)} = (4\pi \times \text{area}) / (\text{circumference} \times \text{circumference})$$

The present invention encompasses dental products fabricated from the alumina workable body for dental use. Examples of such dental products include dental prostheses, orthodontic products, and dental implant products. The dental prostheses are applicable to, for example, alumina inlays, onlays, laminate veneers, and crowns.

The alumina sintered body is obtained by sintering the alumina pre-sintered body or a workpiece thereof. Here, "alumina sintered body" refers to a state where alumina particles (powder) have sintered.

An alumina sintered body of the present invention can be fabricated by sintering the alumina pre-sintered body at a temperature that sinters the alumina particles (sintering step).

The sinterable temperature (for example, highest sintering temperature) is, for example, preferably 1,300°C or more, more preferably 1,350°C or more, even more preferably 1,375°C or more.

The sinterable temperature is, for example, preferably 1,500°C or less, more preferably 1,450°C or less.

The rate of temperature increase to the sinterable temperature, and the rate of temperature decrease from the sinterable temperature are preferably 300°C/min or less.

In the sintering step, the holding time at the sinterable temperature (for example, highest sintering temperature) is preferably 120 minutes or less, more preferably 90 minutes or less, even more preferably 75 minutes or less, yet more preferably 60 minutes or less, particularly preferably 45 minutes or less, most preferably 30 minutes or less. The holding time is preferably 1 minute or more, more preferably 3 minutes or more, even more preferably 5 minutes or more.

The shape and size (dimensions) of the alumina sintered body can be appropriately selected according to factors such as intended use, and the oral environment of patients.

The present invention encompasses embodiments combining the foregoing features in various ways within the technical idea of the present invention, provided that the present invention can exhibit its effects.

In the present invention, the upper limits and lower limits of numeric ranges (for example, ranges of contents of components, ranges of various elements (such as the average primary particle diameter), and ranges of physical properties) can be appropriately combined.

EXAMPLES

The present invention is described below in greater detail through Examples. It should be noted, however, that the present invention is in no way limited by the following Examples, and various changes may be made by a person with ordinary skill in the art within the technical idea of the present invention.

[Fabrication of Alumina Pre-Sintered Body]

<Examples 1 to 7 and Comparative Examples 1 and 3>

Alumina pre-sintered bodies were fabricated using the following procedure to prepare dental alumina pre-sintered bodies for Examples and Comparative Examples.

The following method was used to prepare the raw material powder used for each layer in the fabrication of the alumina pre-sintered bodies of Examples 1 to 7 and Comparative Examples 1 and 3.

First, the sintering aid was measured following the quantities presented in Table 1, relative to 100 g of $\alpha$-aluminaraw material NXA-100 (average primary particle diameter: 100 nm; manufactured by Sumitomo Chemical Co., Ltd.). These were introduced into 1 L of ethanol, and ultrasonically dispersed to prepare a mixture. The mixture was then placed in a rotary container with alumina beads, and crushed by ball milling for 1 hour to obtain a slurry.

After adding an organic binder to the slurry, and the slurry was stirred for 24 hours using rotary vanes. An aqueous acrylic binder, serving as the organic binder, was added in an amount of 2.5 mass% with respect to the $\alpha$-alumina raw material (the organic binder content relative to the entire slurry). After stirring, the slurry underwent drying and granulation with a spray dryer, yielding granules. These granules were used as the raw material powder of each layer in Examples and Comparative Examples.

The method of production of the alumina pre-sintered body is as follows.

First, the raw material powder was filled into a die with inside dimensions of 20 mm $\times$ 25 mm, layering it from bottom to top to provide the lowermost layer, intermediate layer, and uppermost layer. This was followed by uniaxial pressing at 30 MPa and subsequent CIP molding at 200 MPa, producing a molded body with a layered structure. Here, each layer was packed with 7 g of raw material powder. The resulting molded body was placed in an electric furnace, and heated from room temperature at 10°C/min. Following a 2-hour retention at 500°C for the purpose of debinding organic components, the molded body was heated at 10°C/min, maintained for 6 hours at the pre-sintering temperature of Table 1, and gradually cooled at -0.4°C/min to yield an alumina pre-sintered body. In Table 1, the sintering aid content is expressed as ppm by mass in terms of a Mg element.

[Fabrication of Zirconia Pre-Sintered Body]

<Comparative Example 2>

The following method was used to prepare the raw material powder used for the fabrication of the zirconia pre-sintered body of Comparative Example 2. First, a monoclinic zirconia (zirconium oxide) powder and a yttria (yttrium oxide) powder were used to prepare three mixtures with a yttria content of 4 mol%, 5 mol%, and 6 mol% relative to the total mole of zirconia and yttria. After adding water to each mixture to prepare a slurry, the slurry underwent wet pulverization through mixing with a ball mill until an average particle diameter of 0.13 $\mu$m or less was achieved. Alter pulverization, the slurry was dried with a spray dryer, and the resulting powder was fired at 950°C for 2 hours to yield three kinds of powders (primary powders).

These primary powders were individually added into water to prepare slurries, and the slurries underwent wet pulverization through mixing with a ball mill until an average particle diameter of 0.13 $\mu$m or less was achieved. After adding a binder to the pulverized slurries, the slurries were dried with a spray dryer to yield three kinds of powders (secondary powders). The zirconia pre-sintered body of Comparative Example 2 was produced using the same method employed for the fabrication of the alumina pre-sintered body, except for using the secondary powders as raw material powders.

[Appearance Evaluation of Sintered Body]

The alumina pre-sintered bodies and zirconia pre-sintered body of Examples and Comparative Examples were used to fabricate alumina sintered bodies and a zirconia sintered body, using the method described below. The appearance was visually assessed by comparing it with the appearance of natural teeth.

For the assessment, a commercially available shade guide resembling the appearance of natural teeth can be used. A specific example of such a commercially available shade guide is the VITA Classical shade guide manufactured by VITA (Germany) under this trade name.

First, the alumina pre-sintered bodies and zirconia pre-sintered body fabricated using the aforementioned methods were machined into an anterior crown shape using the CAD/CAM system (KATANA® CAD/CAM system, manufactured by Kuraray Noritake Dental Inc.). After machining, the alumina pre-sintered bodies and zirconia pre-sintered body were heated under atmospheric pressure from room temperature to the sintering temperatures of Table 1 at 3°C/min. These were then fired at the sintering temperatures for 2 hours to yield alumina sintered bodies and a zirconia sintered body.

The anterior crown-shaped alumina sintered bodies and zirconia sintered body were visually evaluated using the criteria below.

The alumina sintered bodies and zirconia sintered body were determined as fulfilling the criteria when at least three out of four observers agreed that the following criteria were met. The results are presented in Table 1. Note that the observers observed the alumina sintered bodies and zirconia sintered body from a distance of 30 cm from the eyes.

<Evaluation Criteria>

Good: Demonstrates the same level or better translucency than that of natural teeth, with a sufficient gradation of translucency compared to natural teeth, along with surface reflection comparable to natural teeth.

Moderate: Shows a gradation of translucency, but translucency and/or surface reflection are inferior to natural teeth, or translucency matches or exceeds that of natural teeth but lacks a sufficient gradation when compared to natural teeth.

Poor: Translucency is low, and the gradation of translucency is insufficient compared to natural teeth.

In Examples 1 to 7, all sintered bodies were shown to exhibit the same level or better translucency than that of natural teeth, with a sufficient gradation of translucency compared to natural teeth, and similar surface reflection. This suggests that, with the addition of appropriate coloring components or similar additives, it is possible to faithfully reproduce an appearance comparable to natural teeth.

In contrast, in Comparative Example 1, the translucency was low, and the gradation of translucency was insufficient compared to natural teeth, suggesting that reproduction of an appearance comparable to natural teeth is not possible even with the addition of coloring components or similar additives.

In Comparative Example 2, while the translucency had a gradation, the surface exhibited a glare-like reflection similar to metal surfaces, and there was perceivably weak light transmission in translucency, particularly in the incisal region, suggesting that faithful reproduction of an appearance comparable to natural teeth is not possible.

Comparative Example 3 showed the same level or better translucency than natural teeth; however, the gradation of translucency was insufficient compared to natural teeth. While coloring components or similar additives may diminish translucency by masking the color, it is probably difficult to achieve the shade comparable to natural teeth while sufficiently reducing translucency, and faithfully reproduce an appearance comparable to natural teeth, even with the addition of coloring components or similar additives.

[Measurement Method of Average Crystal Grain Size in Sintered Body]

The average crystal grain size in each layer of the alumina sintered bodies and zirconia sintered body of Examples and Comparative Examples was measured for the alumina sintered bodies and zirconia sintered body individually fabricated for each layer in the manner described in the section [Measurement Method of Total Transmittance and Linear Light Transmittance of Sintered Body].

The measurement was conducted by taking surface images of samples corresponding to each layer of the alumina sintered bodies and zirconia sintered body obtained in Examples and Comparative Examples, using a scanning electron microscope (VE-9800 manufactured by Keyence under this trade name). The crystal grain size was measured by image analysis after indicating grain boundaries on individual crystal grains in the image.

For the measurement of crystal grain size, the captured SEM image was binarized with image analysis software (Image-Pro Plus manufactured by Hakuto Co., Ltd. under this trade name), and particles were recognized from the field (region) by adjusting the brightness range to provide clear grain boundaries.

The crystal grain size from Image-Pro Plus is the average of the measurements of the length of a line segment connecting the contour line and passing through the center of gravity determined from the contour line of the crystal grain, conducted at 2-degree intervals with the center of gravity as the central point. The measurement of crystal grain size was conducted for all particles not extending beyond the edges of the SEM photographic image (3 fields) of each Example and Comparative Example.

The average crystal grain diameter was calculated from the crystal grain size of each particle and the number of crystal grains. The resulting arithmetic average diameter was then determined as the average crystal grain size of the sintered body. Note that particles not extending beyond the edges of the image are particles excluding those with contour lines extending beyond the screen of the SEM photographic image (particles with their contour lines interrupted by the boundary lines at the top, bottom, left, and right). To select the crystal grain size of all particles not extending beyond the edges of the image, the option in Image-Pro Plus was used that excludes all particles lying on the boundary lines. The results are presented in Table 1.

[Measurement Method of Total Transmittance and Linear Light Transmittance of Sintered Body]

The alumina sintered bodies and zirconia sintered body of Examples and Comparative Examples were measured for total transmittance and linear light transmittance in each layer. The measurement was individually conducted for each layer of the alumina sintered bodies and zirconia sintered body fabricated using the following method.

First, a molded body was fabricated from the raw material powder of each layer in Examples and Comparative Examples by pressing the powder in a die having a diameter of 30 mm. Here, the raw material powder was introduced into the die in adjusted amounts to ensure the production of alumina sintered bodies and zirconia sintered body with a thickness of 1.0 mm.

The molded body was heated from room temperature at 10°C/min. Following a 2-hour retention at 500°C for the purpose of debinding organic components, the molded body was heated at 10°C/min, and maintained for 6 hours at the pre-sintering temperatures of Table 1. The molded body was then gradually cooled at -0.4°C/min to yield alumina pre-sintered bodies and a zirconia pre-sintered body.

Subsequently, the alumina pre-sintered bodies and zirconia pre-sintered body were heated at 3°C/min from room temperature to the sintering temperatures presented in Table 1, and fired at the sintering temperatures for 2 hours to yield alumina sintered bodies and a zirconia sintered body.

The alumina sintered bodies and zirconia sintered body were polished to a mirror finish on both sides to prepare alumina sintered bodies and zirconia sintered body with a thickness of 1.0 mm. These alumina sintered bodies and zirconia sintered body were then measured for total transmittance and linear light transmittance with a turbidimeter (Haze Meter NDH 4000 manufactured by Nippon Denshoku Industries Co., Ltd.). The measurement was conducted according to ISO 13468-1 and JIS K 7361-1:1997, and the measured values were averaged for n = 3. The results are presented in Table 1.

[Measurement Method of Biaxial Flexural Strength of Sintered Body]

The alumina sintered bodies and zirconia sintered body of Examples and Comparative Examples were measured for biaxial flexural strength in each layer. The measurement was individually conducted for each layer of the alumina sintered bodies and zirconia sintered body fabricated using the following method.

First, a molded body was fabricated from the raw material powder of each layer in Examples and Comparative Examples by pressing the powder in a die having a diameter of 19 mm. Here, the raw material powder was introduced into the die in adjusted amounts to ensure the production of alumina sintered bodies and zirconia sintered body with a thickness of 1.0 mm.

The molded body was heated from room temperature at 10°C/min. Following a 2-hour retention at 500°C for the purpose of debinding organic components, the molded body was heated at 10°C/min, and maintained for 6 hours at the pre-sintering temperatures of Table 1. The molded body was then gradually cooled at -0.4°C/min to yield alumina pre-sintered bodies and a zirconia pre-sintered body.

Subsequently, the alumina pre-sintered bodies and zirconia pre-sintered body were heated under atmospheric pressure at 3°C/min from room temperature to the sintering temperatures presented in Table 1, and fired at the sintering temperatures for 2 hours to yield alumina sintered bodies and a zirconia sintered body. The alumina sintered bodies and zirconia sintered body were polished to a mirror finish on both sides to prepare alumina sintered bodies and zirconia sintered body with a thickness of 1.0 mm. These alumina sintered bodies and zirconia sintered body were then measured for biaxial flexural strength with a universal testing machine (AG-I 100kN manufactured by Shimadzu Corporation). The measurement was conducted according to JIS T 6526:2012, and the measured values were averaged for n = 5. The results are presented in Table 1.

In Examples 1 to 7, variations in average crystal grain size across three or two layers resulted in a gradation in total transmittance and linear light transmittance while achieving high values in transmittance itself and satisfying the strength required for dental applications. The linear light transmittance was particularly high in the uppermost layer corresponding to the incisal region (the layer including the end P in FIG. 1), indicating superior properties compared to common zirconia materials currently in use.

In contrast, in Comparative Examples 1 and 3, the average crystal grain size was the same across the layers, leading to a lack of gradation in total transmittance and linear light transmittance, and the inability to achieve the same level of translucency as natural teeth. Comparative Example 2 showed a gradation in total transmittance, but the values of linear light transmittance were nearly the same across the layers. Particularly, the incisal region showed a notably low linear light transmittance of 0.7%, failing to achieve the required level of translucency (transmissivity for linear light) in the incisal region.

[Measurement Method of Refractive Index of Sintered Body]

The alumina sintered body of each Example was measured for refractive index in each layer. The measurement was individually conducted for each layer of the alumina sintered body fabricated using the following method.

First, a molded body was fabricated from the raw material powder of each layer in each Example by pressing the powder in a die having inside dimensions of 20 mm $\times$ 25 mm. Here, the raw material powder was introduced into the die in adjusted amounts to ensure the production of alumina sintered bodies with a thickness of 1.0 mm.

The molded body was heated from room temperature at 10°C/min. Following a 2-hour retention at 500°C for the purpose of debinding organic components, the molded body was heated at 10°C/min, and maintained for 6 hours at the pre-sintering temperatures of Table 1. The molded body was then gradually cooled at -0.4°C/min to yield alumina pre-sintered bodies.

Subsequently, the alumina pre-sintered bodies were fired for 2 hours at the sintering temperatures of Table 1 to yield alumina sintered bodies.

After polishing both sides to a mirror finish, the alumina sintered bodies were measured with an Abbe refractometer (DR-M2 manufactured by Atago Co., Ltd. under this trade name), and the measured values were averaged for n = 3. The refractive index was 1.56 in the first layer in Example 1.

From the result yielding a refractive index closer to the refractive index (approximately 1.6) of natural teeth (enamel) compared to zirconia, the light reflection should be comparable to that seen in natural teeth.

] [Measurement Method of Average Roundness of Pre-Sintered Body]

The alumina pre-sintered bodies and zirconia pre-sintered body of Examples and Comparative Examples were used to prepare surface images, using a scanning electron microscope (VE-9800 manufactured by Keyence under this trade name).

For the measurement of the average roundness of particles contained in the pre-sintered bodies, the SEM image with indicated primary particles was binarized after indicating grain boundaries on individual crystal grains in the image, and particles were recognized from the field (region) using image analysis software (Image-Pro Plus manufactured by Hakuto Co., Ltd. under this trade name).

In areas with unclear grain boundaries, a reduce filter was applied to the region until each region reduced to a single or multiple points, and Voronoi polygons were created by using these points as generators of Voronoi polygons. Lines were drawn that connect the midpoints between two adjacent generators, and these lines were superimposed on the original particle image to separate adjacent particles. For example, in the case where image processing produced a particle that looks like a gourd, the particle was separated into two by assuming that two adjoining circular particles are seen as a single particle. In a processing file recognizing primary particles, "Diameter" was selected from "Count/size dialog" to find the distribution. The measurement was conducted for each of four fields in a sample, using the image analysis software (Image-Pro Plus). The resulting data was used to determine the average roundness by calculating roundness using to the following formula (n = 4).

$$\text{(Roundness)} = (4\pi \times \text{area}) / (\text{circumference} \times \text{circumference})$$

[Table 1]

| | | Ex. 1 | Ex. 2 | Ex. 3 | Ex. 4 | Ex. 5 | Ex. 6 | Ex. 7 | Com. Ex. 1 | Com. Ex. 2 | Com. Ex. 3 |
|---|---|---|---|---|---|---|---|---|---|---|---|
| Base material | | Alumina | Alumina | Alumina | Alumina | Alumina | Alumina | Alumina | Alumina | Zirconia | Alumina |
| Average crystal grain size after sintering ($\mu$m) | Uppermost layer | 0.8 | 1.5 | 1.1 | 1.7 | 1.3 | 1.3 | 0.8 | 2.8 | 2.3 | 0.8 |
| | Intermediate layer | 1.4 | 1.8 | 1.8 | 2.2 | 1.5 | 1.5 | 1.4 | 2.8 | 1.0 | 0.8 |
| | Lowermost layer | 2.2 | 2.4 | 2.5 | 2.5 | 2.1 | 2.3 | 1.4 | 2.8 | 0.6 | 0.8 |
| Type of sintering aid | | $MgCl_2$ | $MgCl_2$ | $MgCl_2$ | $MgCl_2$ | $Mg(NO_3)_2$ | $C_4H_6MgO_4$ | $MgCl_2$ | - | - | $MgCl_2$ |
| Sintering aid content[1) (ppm) | Uppermost layer | 1000 | 700 | 1300 | 750 | 1000 | 1000 | 1000 | 0 | 0 | 1000 |
| | Intermediate layer | 700 | 500 | 600 | 300 | 700 | 700 | 700 | 0 | 0 | 1000 |
| | Lowermost layer | 150 | 100 | 20 | 50 | 150 | 150 | 700 | 0 | 0 | 1000 |
| Yttria content (mol%) | Uppermost layer | - | - | - | - | - | - | - | - | 6.0 | - |
| | Intermediate layer | - | - | - | - | - | - | - | - | 5.0 | - |
| | Lowermost layer | - | - | - | - | - | - | - | - | 4.0 | - |
| Total transmittance after sintering (%) (t = 1.0 mm) | Uppermost layer | 73.0 | 48.6 | 52.7 | 43.8 | 65.6 | 65.0 | 73.0 | 29.7 | 50.1 | 73.0 |
| | Intermediate layer | 48.6 | 41.4 | 41.4 | 35.5 | 47.1 | 49.0 | 48.6 | 29.7 | 47.2 | 73.0 |
| | Lowermost layer | 33.3 | 32.2 | 30.9 | 30.5 | 39.7 | 42.8 | 48.6 | 29.7 | 43.7 | 73.0 |

EP 4 438 027 A1

(continued)

| | | Ex. 1 | Ex. 2 | Ex. 3 | Ex. 4 | Ex. 5 | Ex. 6 | Ex. 7 | Com. Ex. 1 | Com. Ex. 2 | Com. Ex. 3 |
|---|---|---|---|---|---|---|---|---|---|---|---|
| Linear light transmittance after sintering (%) (t = 1.0 mm) | Uppermost layer | 11.2 | 6.2 | 6.5 | 5.4 | 8.5 | 10.8 | 11.2 | 0.5 | 0.7 | 11.2 |
| | Intermediate layer | 6.2 | 4.5 | 4.3 | 2.1 | 5.0 | 5.5 | 5.6 | 0.5 | 0.7 | 11.2 |
| | Lowermost layer | 1.2 | 1.1 | 0.9 | 0.8 | 1.1 | 1.0 | 5.6 | 0.5 | 0.6 | 11.2 |
| Biaxial flexural strength after sintering (t = 1.0 mm) MPa | Uppermost layer | 428 | 551 | 341 | 497 | 433 | 454 | 428 | 620 | 530 | 428 |
| | Intermediate layer | 561 | 624 | 610 | 632 | 554 | 568 | 561 | 620 | 753 | 428 |
| | Lowermost layer | 854 | 912 | 720 | 705 | 804 | 821 | 561 | 620 | 897 | 428 |
| Appearance evaluation | | Good | Good | Good | Good | Good | Good | Good | Poor | Moderate | Moderate |
| Pre-sintering temperature[2] (°C) | | 800 | 1000 | 800 | 900 | 800 | 800 | 800 | 800 | 1000 | 800 |
| Sintering temperature under atmospheric pressure[3] (°C) | | 1375 | 1375 | 1375 | 1450 | 1450 | 1375 | 1375 | 1450 | 1550 | 1375 |

1) Sintering aid content means a value in terms of an element (for example, in terms of a Mg element when sintering aid is $MgCl_2$.

2) Highest pre-sintering temperature
3) Highest sintering temperature

EP 4 438 027 A1

**EP 4 438 027 A1**

The average roundness was 0.86 in the first layer of Example 1 whereas it was 0.75 in the first layer of Comparative Example 2, indicating that the alumina pre-sintered body of Example 1 possesses good polishability. The low average roundness in the zirconia pre-sintered body of Comparative Example 2 suggests that this zirconia pre-sintered body is inferior in terms of polishability.

INDUSTRIAL APPLICABILITY

An alumina workable body for dental use of the present invention is useful as dental products. Particularly, an alumina workable body for dental use of the present invention can be suitably used as a dental prosthesis for incisors with the incisal region (central incisors and lateral incisors).

With an alumina workable body for dental use of the present invention, an alumina workable body for dental use can be provided that enables the production of prostheses with an appearance close to natural teeth, including the incisal region. This is achieved by providing high linear light transmittance in portions corresponding to the incisal region while lowering linear light transmittance in portions corresponding to the cervical region, allowing transparency to vary in different locations and producing a gradation in linear light transmittance while ensuring high strength in portions corresponding to the cervical region.

Reference Signs List

| 10 | Alumina workable body for dental use |
| A | First point |
| B | Third point |
| C | Second point |
| P | One end |
| Q | Other end |
| X | Entire length |
| Y | First direction |

**Claims**

1. An alumina workable body for dental use with D1 and D2 that are different from each other, where D1 is the post-sintering average crystal grain size at a first point falling within an interval from one end P to 25% of the entire length on a straight line extending along a first direction from said one end P to the other end Q of the alumina workable body for dental use, and D2 is the post-sintering average crystal grain size at a second point falling within an interval from said other end Q to 25% of the entire length.

2. The alumina workable body for dental use according to claim 1, wherein at least two of the D1, D2, and D3 are different when D3 is the post-sintering average crystal grain size at a third point falling between the first point and the second point on a straight line extending along the first direction from one end P to the other end Q of the alumina workable body for dental use.

3. The alumina workable body for dental use according to claim 1 or 2, which shows an unchanging pattern of increase or decrease in post-sintering average crystal grain size from one end P to the other end Q of the alumina workable body for dental use on a straight line extending along the first direction from one end P to the other end Q of the alumina workable body for dental use.

4. The alumina workable body for dental use according to any one of claims 1 to 3, wherein the D1 is 0.3 $\mu$m or more and 3.0 $\mu$m or less.

5. The alumina workable body for dental use according to any one of claims 1 to 4, wherein the D2 is 1.0 $\mu$m or more and 8.0 $\mu$m or less.

6. The alumina workable body for dental use according to any one of claims 1 to 5, wherein the difference between the D1 and the D2 is 0.3 $\mu$m or more.

7.  The alumina workable body for dental use according to any one of claims 1 to 6, which has a linear light transmittance of 0.8% or more and a biaxial flexural strength of 200 MPa or more at the first point after sintering.

8.  The alumina workable body for dental use according to any one of claims 1 to 7, which has a linear light transmittance of 6.0% or less and a biaxial flexural strength of 400 MPa or more at the second point after sintering.

9.  The alumina workable body for dental use according to any one of claims 1 to 8, which comprises a sintering aid at the first point.

10. The alumina workable body for dental use according to any one of claims 1 to 9, wherein the content of the sintering aid is different at the first point and the second point.

11. The alumina workable body for dental use according to any one of claims 1 to 10, wherein the content of the sintering aid shows an unchanging pattern of increase or decrease from said one end P to said other end Q.

12. The alumina workable body for dental use according to any one of claims 9 to 11, wherein the sintering aid comprises at least one element selected from the group consisting of a Group 2 element, Ce, Zr, and Y

13. The alumina workable body for dental use according to claim 12, wherein the Group 2 element is Mg.

14. The alumina workable body for dental use according to any one of claims 1 to 13, wherein the content of the sintering aid is 30 ppm or more and 3,000 ppm or less at the first point.

15. The alumina workable body for dental use according to any one of claims 1 to 14, wherein the content of the sintering aid is 0 ppm or more and 2,000 ppm or less at the second point.

16. The alumina workable body for dental use according to any one of claims 1 to 15, which comprises a plurality of layers with different average crystal grain sizes after sintering.

17. The alumina workable body for dental use according to claim 16, wherein the plurality of layers has the smallest post-sintering average crystal grain size in the layer including said one end P.

18. The alumina workable body for dental use according to claim 16 or 17, wherein the plurality of layers has the largest post-sintering average crystal grain size in the layer including said other end Q.

19. The alumina workable body for dental use according to any one of claims 1 to 18, wherein the alumina workable body for dental use is an alumina pre-sintered body.

20. The alumina workable body for dental use according to any one of claims 1 to 19, which comprises alumina particles with an average roundness of 0.81 or more in primary particles of the alumina pre-sintered body.

FIG.1

## INTERNATIONAL SEARCH REPORT

| International application No. |
|---|
| **PCT/JP2022/046499** |

| A. | CLASSIFICATION OF SUBJECT MATTER |
|---|---|

*A61K 6/802*(2020.01)i; *A61C 5/70*(2017.01)i; *A61C 13/083*(2006.01)i; *C04B 35/111*(2006.01)i
FI:    A61K6/802; A61C5/70; A61C13/083; C04B35/111

According to International Patent Classification (IPC) or to both national classification and IPC

| B. | FIELDS SEARCHED |
|---|---|

Minimum documentation searched (classification system followed by classification symbols)

A61K6/802; A61C5/70; A61C13/083; C04B35/111

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2004-26513 A (NATIONAL INSTITUTE OF ADVANCED INDUSTRIAL & TECHNOLOGY) 29 January 2004 (2004-01-29)<br>claims, paragraphs [0001], [0010], examples, tables 1-3, fig. 1-2 | 1-20 |
| X | JP 2004-307239 A (NATIONAL INSTITUTE OF ADVANCED INDUSTRIAL & TECHNOLOGY) 04 November 2004 (2004-11-04)<br>claims, paragraphs [0001], [0012], examples, table 1, fig. 1-2 | 1-20 |
| A | JP 2001-213664 A (SUMITOMO CHEMICAL CO., LTD.) 07 August 2001 (2001-08-07)<br>claims, examples | 1-20 |
| A | JP 2005-514305 A (3M INNOVATIVE PROPERTIES COMPANY) 19 May 2005 (2005-05-19)<br>claims, examples | 1-20 |
| A | JP 2019-112271 A (SHOWA DENKO K.K.) 11 July 2019 (2019-07-11)<br>claims, examples | 1-20 |
| A | US 2022/0055948 A1 (IVOCLAR VIVADENT AG) 24 February 2022 (2022-02-24)<br>claims, examples | 1-20 |

☐ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 March 2023** | **20 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/046499**

| Patent document cited in search report | | | Publication date (day/month/year) | Patent family member(s) | | Publication date (day/month/year) |
|---|---|---|---|---|---|---|
| JP | 2004-26513 | A | 29 January 2004 | US 2004/0033394 A1 claims, paragraphs [0002], [0026], examples, tables 1-3, fig. 1-2 EP 1375447 A1 | | |
| JP | 2004-307239 | A | 04 November 2004 | (Family: none) | | |
| JP | 2001-213664 | A | 07 August 2001 | (Family: none) | | |
| JP | 2005-514305 | A | 19 May 2005 | US 2003/0125189 A1 claims, examples WO 2003/057065 A1 EP 1458304 A1 CA 2470415 A1 CN 1607926 A | | |
| JP | 2019-112271 | A | 11 July 2019 | US 2020/0001432 A1 claims, examples WO 2019/131817 A1 EP 3568448 A1 KR 10-2019-0102303 A CN 110382656 A TW 201930231 A | | |
| US | 2022/0055948 | A1 | 24 February 2022 | EP 3957618 A1 | | |

Form PCT/ISA/210 (patent family annex) (January 2015)

**EP 4 438 027 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017185163 A
- WO 2019131782 A
- WO 2020138316 A
- JP 2005514305 T
- JP 2001213664 A